(19) Europäisches Patentamt
European Patent Office
Office européen des brevets

(11) **EP 4 324 403 B1**

(12) **EUROPEAN PATENT SPECIFICATION**

(45) Date of publication and mention
of the grant of the patent:
**29.10.2025 Bulletin 2025/44**

(21) Application number: **23460005.4**

(22) Date of filing: **23.02.2023**

(51) International Patent Classification (IPC):
***A61B 10/00*** (2006.01)   ***C12Q 1/04*** (2006.01)
***G01N 33/493*** (2006.01)

(52) Cooperative Patent Classification (CPC):
**A61B 10/007; B01L 3/508; C12Q 1/04;**
**G01N 33/5091; G01N 33/56911; G01N 33/6893;**
B01L 2300/042; B01L 2300/163; G01N 33/493;
G01N 2800/348

(54) **DIAGNOSTIC KIT FOR DIAGNOSIS OF BACTERIAL URINARY TRACT INFECTIONS (EN)**

DIAGNOSEKIT ZUR DIAGNOSE BAKTERIELLER HARNWEGSINFEKTIONEN (DE)

KIT DE DIAGNOSTIC POUR LE DIAGNOSTIC DES INFECTIONS BACTÉRIENNES DES VOIES URINAIRES (FR)

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **17.08.2022 EP 22190821**

(43) Date of publication of application:
**21.02.2024 Bulletin 2024/08**

(73) Proprietor: **Gdanski Uniwersytet Medyczny
80-210 Gdansk (PL)**

(72) Inventors:
• **Bryl, Ewa Dorota
80-299 Gdansk (PL)**
• **Daca, Agnieszka Elzbieta
83-000 Pruszcz Gdanski (PL)**
• **Jarzembowski, Tomasz Artur
80-288 Gdansk (PL)**
• **Piechowicz, Lidia
80-376 Gdansk (PL)**
• **Witkowski, Jacek
80-299 Gdansk (PL)**
• **Wisniewska, Katarzyna
80-180 Gdansk (PL)**

(74) Representative: **Godlewski, Piotr
FGGH IP
Kancelaria Patentowa Piotr Godlewski
Al. Rzeczypospolitej 20/171
02-972 Warszawa (PL)**

(56) References cited:
PL-B1- 228 911          US-A- 3 563 859
US-A1- 2010 079 751

• DACA AGNIESZKA ET AL: "Changes of urine isolates of Escherichia coli and Klebsiella pneumoniae biofilm affect monocytes' response", WORLD JOURNAL OF MICROBIOLOGY AND BIOTECHNOLOGY, SPRINGER NETHERLANDS, DORDRECHT, vol. 37, no. 11, 28 September 2021 (2021-09-28), XP037579095, ISSN: 0959-3993, [retrieved on 20210928], DOI: 10.1007/S11274-021-03150-Y

**Description**

The invention concerns a diagnostic kit for diagnosing bacterial urinary tract infections, resistant to pre-laboratory errors.

Urinary tract infections (UTIs) are one of the most common infectious diseases in humans. They are estimated to represent approximately 20% of all out-of-hospital infections and 40-50% of hospital-acquired infections. Certain group of people suffering from urinary tract infections develops generalised infection and sepsis. Currently, more than 4 million people in Poland suffer from kidney diseases, with more than 100 people per population of 1,000,000 starting their kidney-replacement therapy each year.

Patients suffering from chronic kidney disease (CKD) represent the group particularly exposed to the risk of urinary tract infections. By suppressing their immunity, CKD increases their susceptibility to infections, urinary tract infections included. The occurrence rate of this disorder results from the high occurrence rate of civilization diseases, primarily diabetes, arterial hypertension, and atherosclerosis.

The most common clinical form of UTI is asymptomatic bacteriuria (AB) which is a problem encountered by many physicians in their daily practice. Initially, it was usually believed that each case of AB should be subject to antibacterial treatment. The opinion prevailing today is that asymptomatic bacteriuria should only be treated in those patients who benefit from the treatment. Hence, before introducing antibacterial treatment of AB, physicians should always consider if the potential benefits of the therapy outweigh the potential negative consequences of using antibiotics or chemotherapeutic agents. The UTI occurrence rate is also higher in pregnant women because of the physiological changes in their organisms during the pregnancy, and ranges between 2% and 11%.

Today, the diagnostics of urinary tract infections is based on determining the number of bacteria in urine based on culturing the microorganisms on microbiological beds and identifying them. As such, the method is sensitive to pre-laboratory errors which affect the multiplication of bacteria in the clinical material and which may result from incorrect sampling, transporting, or storing the material for testing (here: urine). Also, the method used currently requires consideration of a number of clinical parameters in test interpretation, such as the patient's anatomical anomalies or any coexisting diseases.

Known from patent description PL228911B1 is a diagnostic test for identification of the pathogenic potential of *Enterococcus* strains isolated from urine based on activation of a reference monocyte line, where at stage one of the test a biofilm of the tested bacteria is formed on the assay plate in the BHI broth, at the temperature of 37°C, for 72 h and at forced movement of the bed. The obtained biofilm is then flushed with physiological saline, following which added onto the plate with the biofilm is suspension of the monocytes obtained from the culture and suspended in the appropriate medium, and the sample is incubated at 37°C for 60 min., whereupon the suspension from above the biofilm is collected for further testing. The last stage of the test consists in examining the changes of monocyte morphology caused by contact with the bacterial biofilm and assessing the reduction in the number of monocytes which remain in the suspension.

Disclosed in the description of invention US3563859A is a device for testing for the presence and quantity of bacterial colonies comprises a culture media support having a plurality of recesses wherein the media are secured. The media support, which is preferably a transparent plastic rectangular element, is adapted to be engaged by a rectangular container or housing which has guides formed on its inner sidewalls to accommodate the support or insert. To enclose the assembly, an apertured cap or cover is provided, which seats on the rectangular container to form an air-light seal with the housing.

Disclosed in the description of invention US2010/079751A1 is a liquid testing assembly for testing a liquid, the assembly comprising a test vessel and a stopper adapted to fit into a free end of the vessel. The stopper substantially hermetically seals the test vessel from the ambient. Further, the assembly includes a support coated with one or more identifying materials for identifying one or more constituents of the liquid. The support is fixed in the stopper and/or the vessel and extends into its interior for a predetermined distance. The liquid testing assembly when assembled is pre-evacuated to a predetermined vacuum sufficient to draw a predetermined volume of liquid to be sampled into the test vessel. The predetermined volume is of such an amount that it wets the one or more identifying materials ensuring identification of one or more constituents present in the liquid.

Publication Daca Agnieszka et. al.: "Changes of urine isolates of Escherichia coli and Klebsiella pneumoniae biofilm affect monocytes' response", World Journal of Microbiology and Biotechnology, Springer Netherlands, Dordrecht, vol. 37, no. 11, 28 September 2021 (2021-09-28) disclosed studies of biofilm formed in different urinary tract diseases and their impact on monocytes' adherence and activation. Changes in the size and granularity of monocytes, as well as their adherence to biofilm, were assessed using FACSVerse flow cytometer after 1 h co-incubation of monocytes and bacterial biofilm in 37 °C. The obtained results were validated against monocytes incubated without bacteria. The isolates from patients with chronic kidney disease formed the most adherent biofilm regardless the presence or absence of inflammatory reaction. Adherence of monocytes also increased during therapy with immunosuppressive agents, but monocytes' response was different when cyclosporine or tacrolimus were used.

The Creators of the invention conducted research aimed at improving the diagnostic test so that it could be used for testing the pathogenic potential of a broader spectrum of bacteria than that of the *Enterococcus* genus in diagnostic kits for testing urine when suspecting a urinary tract infection.

The solution according to the invention - a diagnostic test which may be used for qualitative microbiological tests of urine, especially in the group of patients particularly exposed to the UTI development and complications.

The invention concerns a diagnostic kit for diagnosing urinary tract bacterial infections as defined in claim 1, especially in the group of patients particularly exposed to the UTI development and complications.

In one of the variants of the solution according to the invention, a test coupon coated with poly-L-lysine is placed in each of the slits (4,5) of the container (1).

Preferably, the outer surface of the walls of the container (1) bears colour markings in the form of lines at the level of 3.5 cm (minimum sample level) and 5 cm (maximum sample level).

The diagnostic test according to the invention ensures substantial reduction of pre-laboratory errors and enables simplified interpretation of urine test results.

The principle of testing samples using the kit according to the invention is based on the method developed by the Creators (PL228911B1) of grouping bacteriuria into the following categories: the one burdened with a high risk of development of invasive infections, and the other one resulting from contamination of the lower section of urethra, which should be deemed a physiological phenomenon.

The invention further concerns a method of *in vitro* diagnosing of urinary tract bacterial infections in a urine sample according to claim 4.

**Table 1**

| Assessment of the results based on the W value which denotes changes in the monocyte (effector) volume at a specific light intensity measured in the axis of the ray falling perpendicular to the assayed cell by one of the detectors of the flow cytometer, FSC - Forward Scatter. W is calculated as the ratio of the forward scatter parameter FSC' at specific light intensity measured in the axis of the ray falling perpendicular to the assayed cell after contact with the biofilm per the forward scatter parameter FSC before contact with the biofilm according to the following formula: $W = FSC/FSC'$; O value [change of monocyte suspension inoculum size: $n/60$ - before contact with the biofilm, $n'/60$ - following contact with the biofilm, inoculum size - n of cells /ml] | |
| --- | --- |
| W value [$W = FSC'/FSC$] O value [$O = (n'/60s)/(n/60s),$] | Result interpretation |
| $W < 1.2$ $O < 1.2$ | Positive: uropathogenic strain infection, urine with symptomatic bacteriuria |
| $W > 1.2$ $0 < 1.2$ | Positive: uropathogenic strain infection, urine with symptomatic bacteriuria |
| $W > 1.2$ $O > 1.2$ | Negative: contamination, urine with asymptomatic bacteriuria |
| $W < 1.2$ $O > 1.2$ | Negative: contamination, urine with asymptomatic bacteriuria |

The enterococci colonising the urinary tract produce extracellular mucus which, together with other pathogens, forms a community of microorganisms called biofilm. Because of the diagnostic problem of differentiating between a physiological biofilm and a pathogenic biofilm, a method was developed to differentiate the biofilm based on the assessment of changes in the morphology of monocytes caused by contact with a bacterial biofilm which forms on the coupon and an assessment of the number of monocytes which remain in the suspension. Since the biofilm makes monocytes adhere to the biofilm surface, the quantity of monocytes in the suspension following contact with the biofilm goes down.

It was established during the research that the THP-1 reference monocyte line exposed to the biofilm formed on the coupon by the bacteria present in urine demonstrated different reactions in contact with the biofilm in the case of symptomatic bacteriuria and in the case of urine for which the test result was negative when using the currently applied method. The difference was observed for frequently isolated microorganisms of the following bacteria species: *Escherichia coli, Klebsiella pneumoniae, K. oxytoca, Proteus mirabilis, Pseudomonas aeruginosa, Acinetobacter sp., Enterococcus faecalis* and *Staphylococcus aureus.*

The solution according to the invention is advantageous in that the poly-L-lysine coat on the coupon improves biofilm adhesion to the coupon, which in turn enables diagnosing urinary tract infections for a larger spectrum of microorganism, thanks to which testing accuracy improves substantially.

Under the influence of bacteria deemed an aetiological factor of urinary tract infections, selected phenotypic properties of monocytes, namely the change in the size of monocytes also referred to as effectors marked as parameter W, at a specific light intensity measured in the axis of the ray falling perpendicular to the assayed cell by one of the detectors of a flow cytometer, FSC, proved lower than those identified following contact with the biological material accumulating on the diagnostic coupon in the case of urine samples in which no symptomatic bacteriuria was identified. Adhesion of the monocytes marked as parameter O to the biofilm surface was higher in the first case, while the sensitivity and specificity of the method when assaying the strain characteristics based on both parameters (W and O), as well as the method's predictive value assessed on the PQStat platform stood at > 90%.

In the solution according to the invention, using a single coupon is sufficient to run diagnostics of urinary tract infections.

Alternatively, using a kit according to the invention incorporating two coupons coated with poly-L-lysine makes it possible to repeat the test twice on a single urine sample, which further increases sensitivity of the test.

The solution according to the invention makes it possible to eliminate pre-laboratory errors caused by contaminating urine samples when processing them before the test, since the biofilm is formed in the container to which the urine sample was collected.

Application of the diagnostic kit according to the invention enables more accurate and faster detection of urinary tract infections and as the result substantially reduces the costs related to the treatment of urinary tract infections (UTIs) and their complications.

The subject matter of the invention described in its embodiment is shown on figures, where:

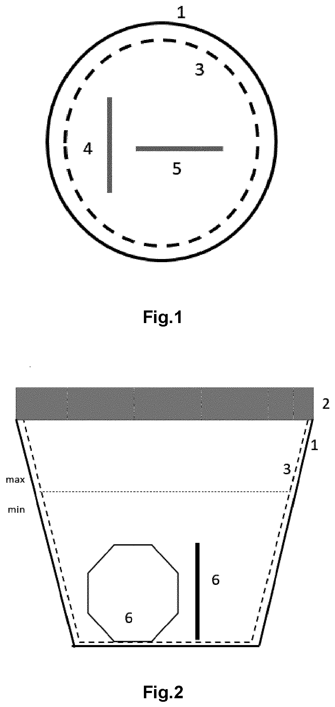

Fig. 1 presents the view of the kit according to the invention from the top, without the cap (2);
Fig. 2 shows a cross section of the kit according to the invention;

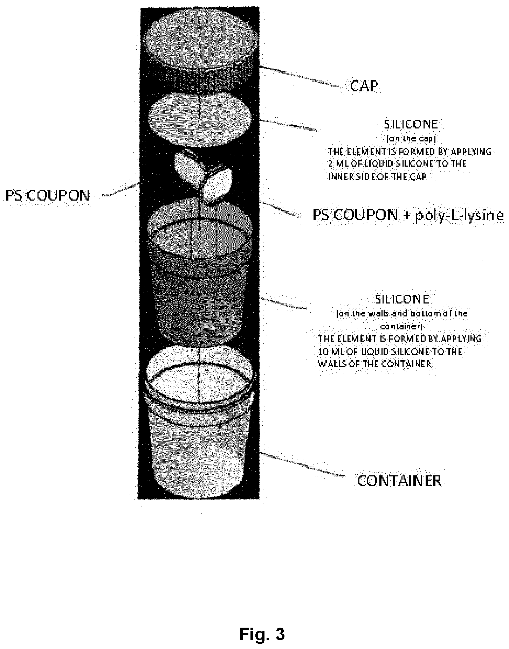

Fig. 3 presents a perspective view of the components making up the diagnostic kit.

The solution according to the invention is illustrated in its embodiment which does not limit its scope.

## Example 1

Inner surface of the walls of a sterile, disposable urine container (1) made of polystyrene was coated with silicone by applying 10 ml of silicone and spreading it into an even layer on the walls, while applied to the inner side of the cap (2) was 2 ml of liquid silicone spread evenly on the entire surface of the cap so that a silicone internal insert (3) is formed once the container is closed; moreover, the insert (3) placed on the bottom of the container (1) was cut to form two slits in which polystyrene coupons were mounted in perpendicular position, where one of the coupons was coated with poly-L-lysine; in addition, colour lines were marked on the outer surface of the walls of the container (1) at the level of 3.5 cm (min) and 5 cm (max), respectively.

## Example 2

**A method of assessing urinary tract infection using the kit according to the invention by the UriMAB method (consisting in monocyte activation caused by a biofilm)** An assay of urine samples collected from patients of the Department of Nephrology, Transplantology and Internal Diseases of the Medical University of Gdańsk was conducted based on approval from the bioethics commission of MUG.

Midstream specimen of urine was collected from each patient to the sterile container (1) incorporating one coupon (6) coated with poly-L-lysine, up to the minimum level marked on the container with the lower line, following which the urine sample in the container (1) was incubated at the temperature of 37°C for 48h at forced movement of the bed, then the coupon (6) was removed from the slit in the container (1), following which the coupon (6) was flushed with physiological saline solution. The obtained biofilm was placed in the cell culture well, added to which was a specific **n** quantity of human reference monocytes of the THP-1 line, suspended in a medium designated for culturing them, in line with the procedure define by the producer of the THP-1 monocyte line (ATCC, *American Type Culture Collection*, TIB-202).

Then, the culture was incubated at the temperature of 37°C for 60 min, whereupon the content of the well was transferred to a cytometer tube.

A flow cytometer was used to determine the number of monocytes detected for 60 s of sample flow, obtaining the parameter **n'/60s.**

Then, change of monocyte inoculum 'O' was calculated for the monocyte suspension using the following formula:

$$O = (n'/60s)/(n/60s)$$

The W and O values were determined for the urine samples

The obtained W and O results were assessed against Table 1 and compared against the reference values obtained, respectively, for microbiologically negative urine samples (asymptomatic bacteriuria) and microbiologically positive urine samples (symptomatic bacteriuria) in terms of the presence of bacteria. The results constituted the basis for assessing the risk of bacteriuria.

The results are presented in Table 2

Table 2

| Sample No. | W Value | O Value | UriMAB method interpretation based on the reference interpretation in Table 1: uropathogenic strain/ contamination | Patient's retrospective data |
|---|---|---|---|---|
| 1 | 0.93 | 0.99 | uropathogenic strain | Symptomatic bacteriuria (>10*6 cfu/ml) of *E. coli* aetiology |
| 2 | 0.92 | 1.12 | uropathogenic strain | Symptomatic bacteriuria (>10*6 cfu/ml), of *K.oxytoca* aetiology |
| 3 | 1.19 | 0.85 | uropathogenic strain | Symptomatic bacteriuria (>10*6 cfu/ml) of *P.aeroginosa* aetiology |
| 4 | 1.2 | 0.79 | uropathogenic strain | Symptomatic bacteriuria (>10*6 cfu/ml) of *S. aureus* aetiology |
| 5 | 1.03 | 0.16 | uropathogenic strain | Symptomatic bacteriuria (>10*6 cfu/ml) of *P.mirabilis* aetiology |
| 6 | 1.05 | 0.74 | uropathogenic strain | Symptomatic bacteriuria (>10*6 cfu/ml) of *E.faecalis* aetiology |
| 7 | 1.24 | 1.85 | contamination | Asymptomatic bacteriuria (<10*6 cfu/ml) *E.coli* |
| 8 | 1.33 | 1.5 | contamination | Asymptomatic bacteriuria (<10*6 cfu/ml) *E.faecalis* |

## Claims

1. A diagnostic kit for diagnosing urinary tract infections, composed of a sterile disposable urine container (1) of a specific volume and a cap (2), wherein the inner surfaces of the walls of the container (1) and the cap (2) are coated with a layer of silicone forming the inner insert (3) of the container (1) and the cap (2), wherein two slits (4, 5) are cut in the silicone insert placed on the bottom of the container, and wherein mounted in at least one of the slits (4, 5), detachably and perpendicularly to the bottom of the container (1), is a polystyrene test coupon (6) coated with poly-L-lysine, and wherein the cap (2) plays the role of a lid of the container (1).

2. The kit according to Claim 1, wherein the container (1) incorporates a diagnostic coupon (6) coated with poly-L-lysine placed in each of the slits (4,5).

3. The kit according to Claims 1 or 2, wherein an outer surface of the walls of the container (1) bears colour markings in the form of lines at the level of 3.5 cm and 5 cm to mark, respectively, the minimum and maximum levels of urine sample.

4. The method of *in vitro* diagnosing of urinary tract bacterial infections in a urine sample, carried out with the kit described in Claims 1-3, wherein

   a. collected to a sterile container (1) containing at least one coupon (6) coated with poly-L-lysine is a midstream specimen of urine, up to the minimum level marked on the container with the lower line,

b. then, the urine sample in the container (1) is incubated at the temperature of 37°C for 48h, at the forced movement of the bed, until a biofilm is formed on the coupon (6),

c. in the next step, the coupon (6) is removed from the slit in the container (1), the coupon (6) is flushed with physiological saline solution and placed in a well for cell culturing, and then a specific quantity of human reference monocytic cells suspended in the medium designated for culturing them, in accordance with the procedure specified by the producer, is added to the well,

d. then, the culture is incubated at the temperature of 37°C for 60 min, following which the content of the well is transferred to a cytometer tube

e. then, a flow cytometer is used to determine the number of monocytes detected over 60s of sample flow (**n'/60s**) and the change in the volume of the monocytes **W** is calculated as the ratio of the forward scatter parameter FSC' at specific light intensity measured in the axis of the ray falling perpendicular to the assayed cell after contact with the biofilm per the forward scatter parameter FSC before contact with the biofilm according to the following formula: W= FSC'/FSC,

f. then, the change of monocyte inoculum size **O** is calculated for the monocyte suspension as the ratio of the number of monocytic cells detected over 60s of sample flow after contact with the biofilm per the number of monocytic cells detected over 60s of flow sample flow before contact with the biofilm according to the following formula: O= (n'/60s)/(n/60s), wherein

for W and O <1.2 or W >1.2 and O <1.2 the result is interpreted as positive and indicative of symptomatic bacteriuria which indicates an infection with a uropathogenic strain;

for W and O >1.2 or W <1.2 and O > 1.2 the result is interpreted as negative and indicative of asymptomatic bacteriuria which indicates contamination.

**Patentansprüche**

1. Ein Diagnostik-Set zur Diagnose von Harnwegsinfektionen, bestehend aus einem sterilen Urinbehälter (1) zur einmaligen Verwendung, mit einem bestimmten Volumen und einer Kappe (2), wobei die Innenflächen der Wände des Behälters (1) und der Kappe (2) mit einer Silikonschicht bezogen sind, die eine innere Einlage (3) des Behälters (1) und der Kappe (2) bildet, wobei zwei Schlitze (4, 5) in der am Boden des Behälters angeordnete Silikoneinlage ausgeschnitten sind, und wobei in mindestens einem der Schlitze (4, 5) ein mit Poly-L-Lysin beschichteter Polystyrol-Teststreifen (6) abnehmbar und senkrecht zum Boden des Behälters (1) angeordnet ist, und wobei die Kappe (2) als Deckel des Behälters (1) fungiert.

2. Das Set gemäß Anspruch 1, wobei
der Behälter (1) einen mit Poly-L-Lysin beschichteten Diagnostikstreifen (6) enthält, der in jedem Schlitz (4, 5) angeordnet ist.

3. Das Set gemäß Anspruch 1 oder 2, wobei
eine Außenfläche der Wände des Behälters (1) mit farbigen Markierungen in Form von Linien in der Höhe von 3,5 cm und 5 cm versehen ist, um den Mindest- bzw. Höchststand der Urinprobe anzuzeigen.

4. Ein Verfahren zur In-vitro-Diagnose von bakteriellen Harnwegsinfektionen anhand einer Urinprobe, das mit dem in den Ansprüchen 1-3 beschriebenen Set durchgeführt wird, wobei

a. eine Probe des Mittelstrahlurins in einen sterilen Behälter (1), der mindestens einen mit Poly-L-Lysin beschichteten Streifen (6) enthält, bis zu dem auf dem Behälter mit der unteren Linie markierten Mindestfüllstand aufgefüllt wird,

b. danach wird die Urinprobe im Behälter (1) bei Temperatur von 37 °C 48 Stunden lang konditioniert, wobei das Bett zwangsweise bewegt wird, bis sich ein Biofilm auf dem Streifen (6) bildet,

c. im nächsten Schritt wird der Streifen (6) aus dem Schlitz im Behälter (1) entnommen, der Streifen (6) wird mit einer physiologischen Kochsalzlösung gespült und in eine Wanne für die Zellkultivierung gelegt, und dann wird eine bestimmte $\pi$-Menge an menschlichen monozytären Referenzzellen, die in dem für ihre Kultivierung bestimmten Medium suspendiert sind, gemäß dem vom Hersteller angegebenen Verfahren in die Wanne eingegeben,

d. anschließend wird die Kultur 60 Minuten lang bei Temperatur von 37 °C konditioniert und der Inhalt der Wanne in einen Zytometer überführt,

e. danach wird ein Durchfluss-Zytometer eingesetzt, um die Anzahl der Monozyten zu bestimmen, die in einem Probenstrom von 60s Dauer (n'/60s) erkannt werden, und die Änderung des Monozyten-Volumens W wird als Verhältnis zwischen den Vorwärtsstreuparametern FSC' bei einer bestimmten Lichtintensität in der Achse des senkrecht auf die untersuchte Zelle fallenden Strahls nach Kontakt mit dem Biofilm und den Vorwärtsstreuparametern FSC vor Kontakt mit dem Biofilm gemäß der folgenden Formel berechnet: W= FSC'/FSC,

f. danach wird die Veränderung des Monozyten-Inokulums O für die Monozyten-Suspension als Verhältnis zwischen der Anzahl der monozytären Zellen, die im 60 Sekunden dauerten Probenstrom nach Kontakt mit dem Biofilm erkannt wurden, und der Anzahl der monozytären Zellen, die im 60 Sekunden dauernden Probenstrom vor dem Kontakt mit dem Biofilm erkannt wurden, nach der folgenden Formel berechnet: O= (n'/60s)/(n/60s), wobei

für W und O <1,2 oder W >1,2 und O <1,2 wird das Ergebnis als positiv und als Hinweis auf eine symptomatische Bakteriurie ausgelegt, die auf eine Infektion mit einem uropathogenen Stamm hinweist; bei W und O >1,2 oder W <1,2 und O > 1,2 wird das Ergebnis als negativ interpretiert und deutet auf eine asymptomatische Bakteriurie hin, die eine Kontamination anzeigt.

## Revendications

1. Un kit de diagnostic pour diagnostiquer les infections des voies urinaires, composé d'un récipient d'urine stérile jetable (1) d'un volume spécifique et d'un bouchon (2), dans lequel les surfaces intérieures des parois du récipient (1) et du bouchon (2) sont recouvertes d'une couche de silicone formant l'insert intérieur (3) du récipient (1) et du bouchon (2), dans lequel deux fentes (4, 5) sont pratiquées dans l'insert en silicone placé sur le fond du récipient, et dans lequel au moins une des fentes (4, 5), montée de manière amovible et perpendiculaire au fond du récipient (1), contient une bandelette d'essai en polystyrène (6) enduite de poly-L-lysine, et dans lequel le bouchon (2) joue le rôle d'un couvercle du récipient (1).

2. Le kit selon la revendication 1, dans lequel
le récipient (1) comprend une bandelette de diagnostic (6) enduite de poly-L-lysine placée dans chacune des fentes (4,5).

3. Le kit selon les revendications 1 ou 2, dans lequel
une surface extérieure des parois du récipient (1) porte des marques de couleur sous forme de lignes au niveau de 3,5 cm et 5 cm pour marquer, respectivement, les niveaux minimum et maximum de l'échantillon d'urine.

4. Méthode de diagnostic in vitro des infections bactériennes des voies urinaires dans un échantillon d'urine, réalisée à l'aide du kit décrit dans les revendications 1 à 3, dans laquelle

a. un échantillon d'urine à mi-parcours, est recueilli dans un récipient stérile (1) contenant au moins une bandelette (6) enduite de poly-L-lysine jusqu'au niveau minimum indiqué sur le récipient par la ligne inférieure,

b. ensuite, l'échantillon d'urine dans le récipient (1) est incubé à la température de 37°C pendant 48h, avec le mouvement forcé du lit, jusqu'à ce qu'un biofilm se forme sur la bandelette (6),

c. à l'étape suivante, la bandelette (6) est retirée de la fente du récipient (1), la bandelette (6) est rincée avec une solution saline physiologique et placée dans un puits pour la culture de cellules, puis une quantité spécifique $\pi$ de cellules monocytaires humaines de référence en suspension dans le milieu désigné pour les cultiver, conformément à la procédure spécifiée par le producteur, est ajoutée au puits,

d. ensuite, la culture est incubée à la température de 37°C pendant 60 min, après quoi le contenu du puits est transféré dans un tube de cytomètrie

e. ensuite, un cytomètre de flux est utilisé pour déterminer le nombre de monocytes détectés sur 60 s d'écoulement de l'échantillon (n'/60 s) et le changement de volume des monocytes W est calculé comme le rapport du paramètre de diffusion vers l'avant FSC' à une intensité lumineuse spécifique mesurée dans l'axe du rayon tombant perpendiculairement à la cellule analysée après contact avec le biofilm par rapport au paramètre de diffusion vers l'avant FSC avant contact avec le biofilm, selon la formule suivante : W= FSC'/FSC,

f. ensuite, la modification de la taille de l'inoculum de monocytes O est calculée pour la suspension de monocytes comme le rapport du nombre de cellules monocytaires détectées sur 60 secondes de flux d'échantillon après contact avec le biofilm par le nombre de cellules monocytaires détectées sur 60 secondes de flux d'échantillon avant contact avec le biofilm, selon la formule suivante : O= (n'/60s)/(n/60s), où

pour W et O < 1,2 ou W >1,2 et O <1,2, le résultat est interprété comme positif et indique une bactériurie symptomatique qui témoigne d'une infection par une souche uropathogène ;
pour W et O >1,2 ou W <1,2 et O >1,2, le résultat est interprété comme négatif et indique une bactériurie asymptomatique qui témoigne d'une contamination.

Fig.1

Fig.2

**Fig. 3**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- PL 228911 B1
- US 3563859 A
- US 2010079751 A1

**Non-patent literature cited in the description**

- Changes of urine isolates of Escherichia coli and Klebsiella pneumoniae biofilm affect monocytes' response. **DACA AGNIESZKA**. World Journal of Microbiology and Biotechnology. Springer Netherlands, 28 September 2021, vol. 37